# EUROPÄISCHE PATENTSCHRIFT

(11) **EP 1 605 927 B1**
(45) Veröffentlichungstag und Bekanntmachung des Hinweises auf die Patenterteilung: **09.01.2008**
(21) Anmeldenummer: 04721079.4
(22) Anmeldetag: 17.03.2004
(51) Int. Cl.: A61K 31/155, A61P 31/04, A61P 31/10

(54) **ANTIMIKROBIEL WIRKENDE GUANIDINDERIVATE AUF BASIS EINES DIAMINS**
ANTI-MICROBIALLY ACTIVE DIAMINE-BASED GUANINE DERIVATIVES
DERIVES DE GUANIDINE A ACTION ANTIMICROBIENNE A BASE D'UNE DIAMINE

(30) Priorität: 20.03.2003 AT 4532003
(43) Veröffentlichungstag der Anmeldung: 21.12.2005
(73) Patentinhaber: Geopharma ProduktionsgmbH, 1210 Wien (AT)
(72) Erfinder: GEORGOPOULOS, Apostolos, A-1190 Wien (AT)
(74) Vertreter: Schwarz, Albin
(86) Internationale Anmeldenummer: PCT/AT2004/000097
(87) Internationale Veröffentlichungsnummer: WO 2004/082671

(56) Entgegenhaltungen:
- EP-A- 0 439 699
- WO-A-01/85676
- FEIERTAG, PETRA ET AL: "Structural characterization of biocidal oligoguanidines" MACROMOLECULAR RAPID COMMUNICATIONS , 24(9), 567-570 CODEN: MRCOE3; ISSN: 1022-1336, 2003, XP001181420

## Beschreibung

Die Erfindung betrifft ein antimikrobiell bzw. mikrobiozid wirkendes Arzneimittel.

Die Entwicklung von neuen Antibiotika befindet sich gerade in den letzten Jahren in einem Wettlauf mit der steigenden Resistenzentwicklung von Mikroorganismen. Die Behandlung von nosokomialen Infektionen, oft verursacht durch resistente Mikroorganismen, stellt eines der größten Probleme von Krankenhäusern weltweit dar. Infektionen im Bereich der Dermatalogie, wie Infektionen nach Hautverbrennungen, Wundinfektionen. Dekubitus oder Akne, benötigen oft längere Antibiotikabehandlungen. Gleichzeitig können Resistenzentwicklungen bei Staphylokokken- und Pseudomonasstämmen häufig beobachtet werden. Auch Infektionen im Magen-Darm-Trakt mit Helikobakter pylori stellen oft ein therapeutisches Problem dar. Darüber hinaus können vor allem Konjunktivitiden, Infektionen im HNO- und Genitalbereich, die bakteriell oder viral bedingt sind, therapeutische Probleme mit sich bringen.

Die Erfindung stellt sich die Aufgabe, ein neues Arzneimittel zur Verfügung zu stellen, welches eine gesteigerte Wirksamkeit gegen eine Vielzahl von Mikroorganismen aufweist.

Diese Aufgabe wird erfindungsgemäß gelöst durch die Verwendung eines polymeren Guanidinderivates auf Basis eines Diamins, welches Oxyalkylenketten zwischen zwei Aminogruppen enthält, wobei das Guanidinderivat ein Produkt der Polykondensation eines Guanidin-Säureadditionssalzes mit einem Diamin, welches Polyoxyalkylenketten zwischen zwei Aminogruppen enthält, darstellt, sowie deren pharmazeutisch akzeptable Salze, zur Herstellung eines antimikrobiell wirksamen Arzneimittels.

Ferner betrifft die Erfindung die Verwendung der Polyoxyalkylen-Guanidin-Salze, hergestellt unter Einsatz von Triethylenglykoldiamin (relative Molekularmasse: 148), von Polyoxypropylendiamin (relative Molekularmasse: 230) sowie von Polyoxyethylendiamin (relative Molekularmasse: 600).

Als Wirkstoff ist besonders bevorzugt Poly-[2-(2-ethoxy-ethoxyethyl)-guanidiniumhydrochlorid] mit mindestens 3 Guanidiniumresten enthalten, wobei die mittlere Molekularmasse insbesondere im Bereich 500 und 3.000 D liegt.

Die erfindungsgemäß eingesetzten polymeren Guanidinderivate sind aus der PCT/AT01/00134 bekannt.

Die Herstellung eines bevorzugten Vertreters der erfindungsgemäß eingesetzten Verbindungen sowie der Nachweis der antimikrobiellen Wirksamkeit werden nachfolgend beschrieben. Stellvertretend für die erfindungsgemäß eingesetzte Verbindungsklasse wird nachfolgend die antimikrobielle Wirksamkeit von Poly-[2-(2-ethoxy-ethoxyethyl)-guanidinium-hydrochlorid] mit einer mittleren Molekularmasse von 1000 D beschrieben (CAS Nr. 374572-91-5).

Zur Herstellung dieser Verbindung wurden 4,43 Mol Guanidiniumhydrochlorid in 4,03 Mol Triethylenglycoldiamin bei 50°C gelöst. Anschließend wurde auf 120°C erwärmt und zwei Stunden bei dieser Temperatur gerührt. Danach wurde die Temperatur 2 Stunden gehalten, dann ein Vakuum (0,1 bar) angelegt und zwei weitere Stunden unter Vakuum bei 170°C gerührt. Anschließend wurde auf Normaldruck belüftet, auf 120°C abkühlen gelassen und mit entmineralisiertem Wasser auf ca. 50% verdünnt. Mit Phosphorsäure wurde auf einen pH von a. 6 neutralisiert, abkühlen gelassen und auf die gewünschte Konzentration verdünnt. Das Molekulargewicht wurde mit 1000 D bestimmt.

Der Wirkstoff Poly-[2-(2-ethoxy-ethoxyethyl)-guanidinium-hydrochlorid] besitzt, bei geringer Toxizität und guter Verträglichkeit aus pharmakologischer Sicht, günstige pharmakodynamische Eigenschaften und kann daher als Heilmittel in der antimikrobiellen Therapie verwendet werden. Insbesondere zeigt der Wirkstoff eine ausgezeichnete antimikrobielle Wirksamkeit, die durch Untersuchungen an einer Vielzahl von Mikroorganismen, wie multiresistenten Bakterien (die gegenüber üblichen Antibiotika resistent sind), Pilzen (Sprosspilze, Dermatophyten, Schimmelpilze) und Viren, wie Herpes simplex, demonstriert werden konnte. Durch die rasche mikrobizide Wirksamkeit ist eine Resistenzentwicklung kaum zu erwarten, wie auch die Untersuchungen an einer größeren Anzahl von Bakterienstämmen (30 Bakterienspezies und 30 Passagen) gezeigt haben.

Nach systemischer Verabreichung (intravenös oder intraperitoneal) von Poly-[2-(2-ethoxy-ethoxyethyl)-guanidinium-hydrochlorid] in Mengen bis zu 15 mg/kg Körpergewicht werden in der Ratte im Blut Serumkonzentrationen bis zu 100 µg/ml nach zwei Stunden gemessen, bei gleichzeitig guter Verträglichkeit. Diese Konzentrationen liegen weit über dem zu erwartenden benötigten Spiegel bei einer therapeutischen Anwendung. Gleichzeitig wurde auch bei dieser hohen Dosierung eine gute Verträglichkeit beobachtet, es kam zu keiner Mortalität oder schwerwiegenden Nebenwirkungen. Daher kann Poly-[2-(2-ethoxy-ethoxyethyl)-guanidinium-hydrochlorid] als antimikrobielles Mittel verwendet werden.

Als Heilmittel kann der Wirkstoff in geeigneter Arzneiform allein oder gemeinsam mit anorganischen oder organischen, pharmakologisch indifferenten Hilfsstoffen verabreicht werden. Beispielsweise kann die Substanz als Bestandteil einer Salbe oder Lösung eingesetzt werden, wobei die Konzentration in der Salbe ca. 0,01 bis 5g Wirkstoff pro g Salbe oder in der Lösung bis zu einer Konzentration von 15 mg/kg Körpergewicht betragen kann. Durch die genannten günstigen pharmakologischen Eigenschaften ist die Anwendung als Salbe oder Lösung bei einer Vielzahl von Indikationen als günstig zu sehen. In Frage kommen dafür besonders Infektionen der Haut, der Schleimhäute, der Augen oder des Magen-Darm-Traktes. In diesen Bereichen kann der erfindungsgemäße Wirkstoff somit einen wesentlichen Beitrag zur Vermeidung von Antibiotika-Resistenzen leisten.

### Antimikrobielle Wirksamkeit

Zur Prüfung auf die antimikrobielle Wirksamkeit wurden 334 Mikroorganismen, klinisch relevante Isolate und ATCC-Stämme als Referenzkeime hinsichtlich ihrer minimalen Hemmkonzentration (MHK) getestet. Weiters wurde die Zeit- und konzentrationsabhängige Abtötung der Keime (killing curves) und darüber hinaus das Potential der Resistenzentwicklung überprüft.

### 1. Mikroorganismen:

Insgesamt wurden 323 Bakterien unterschiedlicher Spezies, 10 Sprosspilze sowie ein Dermatophyt verwendet. Die getesteten Mikroorganismen wurden aus dem Respirationstrakt, dem Urogenitaltralct, aus Blut und anderem biologischen Material von Patienten nach Labor-Standardmethoden isoliert und genau charakterisiert.

### 2. Testsubstanz:

Als Stammlösung wurde das oben beschriebene Poly-[C2-(2-ethoxy)-ethoxyethyl)-guanidium-chlorid] (Molekularmasse: 1000 D) in einer 25%-igen wässrigen Lösung verwendet. Diese wurde mit sterilem Wasser bis zur jeweiligen gebrauchsfertigen Konzentration verdünnt. Die Aufbewahrung der Testsubstanzen erfolgt bei Zimmertemperatur.

### 3. Bestimmung der minimalen Hemmkonzentration (MHK):

Die Überprüfung der Aktivitäten der Testsubstanz erfolgte mittels Mikrodilutions-Methode in Mueller-Hinton Bouillon nach den NCCLS-Vorschriften. Das mikrobielle Inokulum betrug mindestens 5x10⁵ CFU/ml und die Inkubation wurde für 16 bis 20 Stunden bei 36°C/Umgebungsluft durchgeführt. Der Wirkstoff wurde in Konzentrationen von 1000 µg/ml bis 0,001 µg/ml verwendet.

Die niedrigste Wirkstoffkombination, bei der kein bakterielles Wachstum sichtbar war, wurde als MHK definiert. Bei jedem Testansatz wurden ATCC-Qualitätskontrollstämme mit bekannter MHK mitgeführt.

### 4. Resistenzentwicklung:

Auf der Basis der MHK-Werte wurden 30 Bakterienstämme für die Experimente der Resistenzentwicklung verwendet:
Gram-positive Keime: MSSA (n=1), MRSA (n=2), MRSE (n=4), VRE (n=5), S. aureus ATCC 29213.
Gram-negative Keime: multiresistente E. coli (n=4), Klebsiella pneumoniae (n=1), Klebsiella oxytoca (n=1), Pseudomonas aeruginosa (n=4), multiresistente Pseudomonas aeruginosa (n=4), Acinetobacter sp. (n=2) und E. coli ATCC 35218 und 25922.

Alle Stämme wurden mit der Testsubstanz in Konzentrationen von 1000 µg/ml bis 0,001 µg/ml über 24 Stunden inkubiert. Teströhrchen, in welchen Bakterienwachstum nach der ersten Passage festgestellt wurde, wurden als Inokulum für die zweite Passage verwendet. Es wurden für jeden Bakterienstamm 30 Passagen durchgeführt, wobei die neue MHK immer mit der MHK vom Beginn der Experimente (1. Passage), verglichen wurde.

### 5. Zeit- und konzentrationsabhängige Keimabtötung:

Um das mikrobiozide Potential des Wirkstoffs zu erfassen, wurden jeweils zwei multiresistente Stämme von Staphylokokken, Enterokokken, E.coli und Pseudomonas aeruginosa verwendet.

Die Geschwindigkeit der Abtötung wurde nach Zugabe (Zeit 0) der Bakteriensuspension (1x10⁶ bis 5x10⁶ CFU/ml) und nach 2, 5, 10 und 30 Minuten überprüft.

Bei diesen Experimenten wurde eine quantitative Keimreduktion zu jedem Zeitpunkt und bei jeder Konzentration durchgeführt. Der Wirkstoff wurde in den Konzentrationen von 10%, 1%, 0,1% und 0,001% verwendet. Als Kontrolle wurde stets auch eine Reihe ohne Wirkstoff mitgeführt.

### 6. Minimale Hemmkonzentration (MHK):

Die Ergebnisse der MHK-Prüfung sind in den Tabellen 1 bis 5 zusammengefasst dargestellt.

Bei Staphylococcus aureus und Staphylococcus epidermidis zeigte der Wirkstoff unabhängig vom Resistenzprofil der Isolate gegenüber Antibiotika eine sehr gute Wirkung mit MHK-Werten von 4 bis 32 µg/ml. Auch die multiresistenten Staphylokokken (MRSA) zeigten MHKs in diesem Bereich (Tabelle 1).

Ebenso erbrachte die Testung für Enterococcus faecalis MHK-Werte von 16 bis 32 µg/ml (Tabelle 1), wobei sich wiederum die multiresistenten und vancomycinresistenten Enterokokkenstämme (n=5) nicht von den empfindlichen Isolaten unterschieden.

Enterobacteriacae: Gegenüber E.coli, Klebsiella species, Enterobacter species und Proteus mirabilis erbrachte der Wirkstoff sehr gute Ergebnisse mit MHK-Werten von 4 bis 32 µg/ml (Tabelle 2).

Sehr gut erfasst wurden auch die getesteten Salmonellen, Shigellen und Yersinia enterocolitica (Tabelle 3).

Die Nonfermenter-Gruppe, Pseudomonas aeruginosa und Acinetobacter species, erwies sich ebenfalls gegenüber dem Wirkstoff als empfindlich, mit MHK-Werten von 4 bis 32 µg/ml (Tabelle 3).

Auch hier zeigte sich an fünf Pseudomonas-Isolaten, die gegenüber sämtlichen klinisch relevanten Antibiotika resistent waren, die gute Wirksamkeit des erfindungsgemäß eingesetzten Wirkstoffes.

Ebenso wirksam war der Wirkstoff gegenüber Mykobakterium tuberculosis, avium complex, kansaii und gordonae (Tabelle 4) mit MHK-Werten von 16 bis 32 µg/ml.

Die Testung von klinisch relevanten Pilz-Species wie Candida albicans, Cantida tropicalis und Candida parapsilosis (Sprosspilz) sowie Trichophyton mentagrophytes (Dermatophyt) erbrachte ebenfalls eine sehr gute antimykotische Wirkung (MHK-Werte von 8 bis 32 µg/ml) (Tabelle 5).

### 7. Resistenzentwicklung:

Hierbei wurden insgesamt 30 Keime aus neun verschiedenen pathogenen Bakterienspezies - Gram-positive Keime, wie Staphylococcus aureus und epidermidis, sowie Gram-negative Keime wie E.coli, Klebsiella spp., Pseudomonas aeruginosa und Acinetobacter spp. - verwendet. Getestet wurden sowohl empfindliche ATCC-Stämme, als auch multiresistente klinische Isolate.

Bei beiden Gruppen konnte nach 30 Passagen keine Resistenzentwicklung beobachtet werden, d.h. es wurde keine Erhöhung der MHK-Werte festgestellt.

### 8. Zeit- konzentrationsabhängige Keimabtötung:

Um die bakterizide Eigenschaft des Wirkstoffes zu evaluieren, wurde die Geschwindigkeit der Abtötung der Bakterien bestimmt.

Für die Experimente wurden je zwei Stämme von Staphylococcus aureus, Enterococcus faecalis, E.coli und Pseudomonas aeruginosa verwendet.

Bei Staphylococcus aureus, E.coli und Pseudomonas aeruginosa war die Abtötungsfähigkeit des Wirkstoffs bis zu einer Konzentration von 0,01 % unmittelbar nach Gabe des Inokulums gegeben (0 bis 30 Sekunden).

Bei den Enterokokken wurde für die Abtötung der Keime, bei einer Konzentration von 0,01% Wirkstoff, ein Zeitraum von 10 bis 30 Minuten benötigt.

**Tabelle 1. Wirksamkeit gegenüber Gram-positiven Bakterien**

| **Species** | **Anzahl der Stämme n (%) mit einer MHK (in mg/l) von** | | | |
|---|---|---|---|---|
| | **4** | **8** | **16** | **32** |
| **MSSA (n = 35)** | 0 (0) | 6 (17,1) | 20 (57,1) | 9 (25,8) |
| **MRSA (n = 60)** | 3 (5) | 10 (16,7) | 38 (63,3) | 9 (15) |
| **Enterococcus faecalis (n = 32)** | 0 (0) | 0 (0) | 16 (50) | 16 (50) |

**Tabelle 2. Wirksamkeit gegenüber Enterobacteriacae**

| **Species** | **Anzahl der Stämme n (%) mit einer MHK (in mg/l) von** | | | |
|---|---|---|---|---|
| | **4** | **8** | **16** | **32** |
| **Klebsiella spp. (n = 43)** | 0 (0) | 5 (11,6) | 18 (41,9) | 20 (46,5) |
| **Escherichia coli (n = 59)** | 2 (3,4) | 17 (28,8) | 39 (66,1) | 1 (1,7) |
| **Enterobacter spp. (n = 17)** | 1 (5,9) | 1 (5,9) | 12 (70,6) | 3 (17,6) |
| **Proteus spp. (n = 7)** | 0 (0) | 0 (0) | 3 (42,9) | 4 (57,1) |

**Tabelle 3. Wirksamkeit gegegenüber Gram-negativen Bakterien**

| **Species** | **Anzahl der Stämme n (%) mit einer MHK (in mg/l) von** | | | |
|---|---|---|---|---|
| | **4** | **8** | **16** | **32** |
| **Pseudomonas spp.. (n = 55)** | 1 (1,8) | 5 (9,1) | 32 (58,2) | 17 (30,9) |
| **Acinetobacter spp. (n = 2)** | 0 (na) | 0 (na) | 1 (na) | 1 (na) |
| **Salmonella spp. (n = 6)** | 0 (na) | 2 (na) | 4 (na) | 0 (na) |
| **Shigella spp. (n = 2)** | 0 (na) | 0 (na) | 1 (na) | 1 (na) |
| **Yersinia enterocolitica (n = 1)** | 0 (na) | 0 (na) | 0 (na) | 1 (na) |

**Tabelle 4. Wirksamkeit gegenüber Mykobakterien**

| **Species** | **Anzahl der Stämme n (%) mit einer MHK (in mg/l) von** | | | |
|---|---|---|---|---|
| | **4** | **8** | **16** | **32** |
| **Mykobakterium spp. (n = 6)** | 0 (na) | 0 (na) | 5 (na) | 1 (na) |

**Tabelle 5. Wirksamkeit gegenüber Pilzen**

| **Species** | **Anzahl der Stämme n (%) mit einer MHK (in mg/l) von** | | | |
|---|---|---|---|---|
| | **4** | **8** | **16** | **32** |
| **Candida spp. (n = 10)** | 0 (na) | 1 (na) | 3 (na) | 6 (na) |
| **Trichophyton** m**entagrophytes (n =1)** | 0 (na) | 0 (na) | 1 (na) | 0 (na) |

### Klinische Wirksamkeit

Um die gute in-vitro Aktivität des Wirkstoffes in-vivo zu bestätigen, wurde die Substanz unter klinischen Bedingungen an freiwilligen Patienten mit problematischen Infektionen angewandt. Die Substanz wurde in einer wässrigen Lösung oder in Gel-Form in einer 0,5 %-igen Konzentration verwendet.

### Patienten

Es wurden 12 Patienten im Alter von 35 bis 62 Jahren mit dem Wirkstoff behandelt.
2 Patienten mit chronischer Gastritis (Helicobacter pylori positiv)
3 Patienten mit chronischer Entzündung der Gingiva und der Mundschleimhaut
3 Patienten mit chronischer Entzündung der Vaginalschleimhaut
1 Patient mit Fußgangrän
3 Patienten mit Onichomykose (1 Patient mit Hand-, 2 Patienten mit Fußmykose)

### Ergebnisse

Die klinische Wirksamkeit des Wirkstoffes bei 12 ausgewählten Patienten ist in Tabelle 6 zusammengefasst dargestellt. Ebenso sind die Art der Behandlung, Behandlungsdauer, klinische Leitsymptome und klinische Erfolg ersichtlich.

Kurz zusammengefasst konnte in allen 12 Fällen nach einer Behandlungsdauer von 2 bis 28 Tagen eine überzeugende klinische Wirksamkeit beobachtet werden. Es kam zu einer deutlichen Verbesserung der Leitsymptome bei gleichzeitig ausgezeichneter lokaler Verträglichkeit. Es traten in keinem der Fälle Nebenwirkungen wie Rötungen, Irritationen oder Übelkeit auf.

**Tabelle 6**

| **Anzal der Patienten** | **Indikation** | **klinische Symptome** | **Art der Behandlung** | **Dauer der Behandlung (in Tagen)** | **klinischer Erfolg** |
|---|---|---|---|---|---|
| 2 | chronische Gastritis (H. pylori positiv) | gastrale Druckschmerzen, Sodbrennen | 2x tgl. 0,5% Lösung oral | 14 | keine Beschwerden |
| 3 | Entzündung der Gingiva und der Mundschleimhaut | schmerzhafte Entzündungsherde, Zahnfleischbluten | 2x tgl. 0,5% Lösung lokal | 2 | deutliche Abheilung, keine Schmerzen, kein Zahnfleischbluten |
| 3 | chronische Entzündung der Vaginalschleimhaut | Brennen, Ausfluss, Juckreiz | 2x tgl. 0,5% Lösung lokal | 5 | keine Beschwerden |
| 1 | Fußgangrän | ulceröse Veränderungen, ödemätose Durchdrengung, stinkender Geruch, Operation kontraindiziert | 3x tgl. 0,5% Lösung lokal | 28 | deutliche Besserung des Lokalbefundes, keine Geruchsentwicklung, Operationsvorbereitung eingeleitet |
| 3 | Hand- und Fußmykose | Juckreiz, Nageldeformierung- und Verfärbung | 3x tgl. 0,5% Lösung lokal | 28 | kein Juckreiz, Nachwachsen eines gesunden Nagels |

## Patentansprüche

1. Verwendung eines polymeren Guanidinderivates auf Basis eines Diamins, welches Oxyalkylenketten zwischen zwei Aminogruppen enthält, wobei das Guanidinderivat ein Produkt der Polykondensation eines Guanidin-Säureadditionssalzes mit einem Diamin, welches Polyoxyalkylenketten zwischen zwei Aminogruppen enthält, darstellt, zur Herstellung eines antimikrobiell wirksamen Arzneimittels.

2. Verwendung nach Anspruch 1, **dadurch gekennzeichnet, dass** als Vertreter der Reihe der Polyoxyalkylen-Guanidin-Salze solche unter Einsatz von Triethylenglykoldiamin (relative Molekularmasse: 148), von Polyoxypropylendiamin (relative Molekularmasse: 230) sowie von Polyoxyethylendiamin (relative Molekularmasse: 600) sind.

3. Verwendung nach einem der Ansprüche 1 oder 2, **dadurch gekennzeichnet, dass** als Poly-[2-(2-ethoxy-ethoxyethyl)-guanidinhun-hydrochlorid] mit mindestens 3 Guanidiniumresten eingesetzt wird.

4. Verwendung nach Anspruch 3, **dadurch gekennzeichnet, dass** die mittlere Molekularmasse des Wirkstoffes im Bereich 500 bis 3.000 liegt.

5. Verwendung nach einem der Ansprüche 1 bis 4, **dadurch gekennzeichnet, dass** das Arzneimittel als veterinärmedizinisches Arzneimittel ausgebildet ist.

## Claims

1. The use of a polymeric guanidine derivative based on a diamine containing oxyalkylene chains between two amino groups, with the guanidine derivative representing a product of polycondensation between a guanidine acid addition salt and a diamine containing polyoxyalkylene chains between two amino groups, for the production of a drug composition with antimicrobial activity.

2. The use according to claim 1, **characterized in that**, among the representatives of the family of polyoxyalkylene guanidine salts, there are such using triethylene glycol diamine (relative molecular mass: 148), polyoxypropylene diamine (relative molecular mass: 230) as well as polyoxyethylene diamine (relative molecular mass: 600).

3. The use according to any of claims 1 or 2, **characterized in that** poly-[2-(2-ethoxyethoxyethyl)guanidinium hydrochloride] having at least 3 guanidinium groups is used.

4. The use according to claim 3, **characterized in that** the average molecular mass of the drug substance ranges from 500 to 3.000.

5. The use according to any of claims 1 to 4, **characterized in that** the drug composition is designed as a drug composition for veterinary use.

## Revendications

1. Utilisation d'un dérivé polymère de guanidine à base d'une diamine, qui contient des chaînes oxyalkyle entre deux groupes amino, le dérivé de guanidine représentant un produit de la polycondensation d'un sel d'addition acide de guanidine avec une diamine, qui contient des chaînes de polyoxyalkyle entre deux groupes amino, pour la préparation d'un médicament à effet antimicrobien.

2. Utilisation selon la revendication 1, **caractérisée en ce qu'**à titre de représentant de la série des sels de polyoxyalkyle-guanidine, on prévoit ceux qui utilisent du triéthylèneglycoldiamine (masse moléculaire relative : 148), de polyoxypropylène-diamine (masse moléculaire relative: 230) ainsi que de polyoxyéthylènediamine (masse moléculaire relative : 600).

3. Utilisation selon l'une des revendications 1 ou 2, **caractérisée en ce que** l'on utilise du poly-[2-(2-éthoxy-éthoxyéthyl)-guanidinium-hydrochlorure] avec au moins trois restes de guanidinium.

4. Utilisation selon la revendication 3, **caractérisée en ce que** la masse moléculaire moyenne de la substance active est dans la plage allant de 500 à 3000.

5. Utilisation selon l'une des revendications 1 à 4, **caractérisée en ce que** le médicament est réalisé sous forme de médicament vétérinaire.
